# EUROPEAN PATENT APPLICATION

(11) **EP 4 307 183 A1**
(43) Date of publication of application: **17.01.2024**
(21) Application number: 21930072.0
(22) Date of filing: 09.03.2021
(51) Int. Cl.: G06N 20/00

(54) **MACHINE LEARNING PROGRAM, PREDICTION PROGRAM, DEVICE, AND METHOD**

(71) Applicant: FUJITSU LIMITED, Kawasaki-shi, Kanagawa 211-8588 (JP)
(72) Inventor: UKAI, Takanori, Kawasaki-shi, Kanagawa 211-8588 (JP)
(74) Representative: Hoffmann Eitle
(86) International application number: PCT/JP2021/009344
(87) International publication number: WO 2022/190215

(57) **Abstract**

When a machine learning device updates embedding vectors by adding additional data represented by a triple (22) that includes a first node, a second node, and a first edge that indicates a relationship between the first node and the second node to graph data that includes a plurality of nodes and a plurality of edges that indicates relationships between the plurality of nodes and in which a plurality of embedding vectors that represents the respective plurality of nodes and the respective plurality of edges has been calculated by machine learning, the machine learning device updates, in the graph data, the respective embedding vectors of one or a plurality of nodes and one or a plurality of edges in a range (24) coupled to the triple (22) that represents the additional data under a specific condition.

## Description

### FIELD

The disclosed technology relates to a machine learning technology.

### BACKGROUND

Conventionally, a technology of predicting a specific event or the like by using graph data including a plurality of nodes and a plurality of edges indicating a relationship between the nodes has been proposed. For example, a system for discovery of predicted site-specific protein phosphorylation statements has been proposed. The system uses data from a phosphorylation dataset, a kinase family database, and a protein sequence database that store known site-specific statements to generate motif-based phosphorylation statements. Furthermore, the system generates negative statements from the motif-based statements, generates candidate statements by combination of the motif-based statements, and generates negative candidate statements by combination of the candidate statements with the negative statements. Furthermore, the system uses the candidate statements and the negative candidate statements in a statistical relationship training model to score the candidate statements, and retains predicted motif-based phosphorylation statements. Furthermore, the system receives data input from the protein sequence database and the predicted motif-based phosphorylation statements in a generalized format to generate predicted site-specific phosphorylation statements in a site-specific format.

Furthermore, for example, a device that completes a knowledge graph from a plurality of predicates each providing information regarding a relationship between a pair of entities and associated entities has been proposed. The device receives input including the plurality of predicates and the associated entities, searches an axiom database, and identifies predicates that are equivalent to one another or inverses of one another among the plurality of predicates. Furthermore, the device identifies further predicates that are related to one another by using the axiom database and the identified predicates, and embeds the identified predicates and the associated entities into a vector space to complete the knowledge graph.

Furthermore, for example, a system that generates a recommendation for a user based on a knowledge graph has been proposed. The system collects a dataset including a plurality of entities and attributes for the plurality of entities, generates relationships between the plurality of entities, and stores the plurality of entities, the attributes, and the relationships in the knowledge graph. Furthermore, the system is subjected to training using a representation of the plurality of entities, the attributes, and the relationships stored in the knowledge graph, executes zero-shot learning for a new entity and attributes for the new entity, and stores the new entity and the attributes for the new entity in the knowledge graph. Then, the system generates a recommendation for a user based on the knowledge graph.

### CITATION LIST

### PATENT DOCUMENT

Patent Document 1: Japanese Laid-open Patent Publication No. 2018-206374
Patent Document 2: Japanese Laid-open Patent Publication No. 2018-85116
Patent Document 3: Japanese Laid-open Patent Publication No. 2019-125364

### SUMMARY

### [TECHNICAL PROBLEM]

It is assumed that, by machine learning based on a triple of entities and a relation representing a relationship between the entities included in graph data, an embedding vector representing each of the entities and the relations is generated. In a case where the machine learning is executed again from the beginning on the graph data to which a newly obtained triple is added, in other words, in a case where batch processing is executed, in order to reflect information regarding the newly obtained triple on the graph data, there is a problem that a processing load of the machine learning is high.

As one aspect, an object of the disclosed technology is to reduce a processing load of machine learning of embedding vectors of graph data.

### [SOLUTION TO PROBLEM]

As one aspect, the disclosed technology acquires information regarding a first triple that includes a first node, a second node, and a first edge that indicates a relationship between the first node and the second node. Furthermore, the disclosed technology updates vectors based on the information and a plurality of vectors that represents respective plurality of nodes and respective plurality of edges that indicate relationships between the plurality of nodes, which is generated by machine learning that uses the plurality of nodes and the plurality of edges. The vectors to be updated are respective vectors of one or a plurality of nodes and one or a plurality of edges that are coupled to the first triple under a specific condition among the plurality of vectors.

According to an aspect of the embodiments, a machine learning program that causes a computer to execute a process, the process includes acquiring information regarding a first triple that includes a first node, a second node, and a first edge that indicates a relationship between the first node and the second node; and updating, based on the information and a plurality of vectors that represents respective plurality of nodes and respective plurality of edges that indicate relationships between the plurality of nodes, which is generated by machine learning that uses the plurality of nodes and the plurality of edges, respective vectors of one or a plurality of nodes and one or a plurality of edges that are coupled to the first triple under a specific condition among the plurality of vectors.

### [ADVANTAGEOUS EFFECTS OF INVENTION]

As one aspect, there is an effect that it is possible to reduce a processing load of machine learning of embedding vectors of graph data.

### BRIEF DESCRIPTION OF DRAWINGS

FIG. 1 is a diagram illustrating an example of case data;
FIG. 2 is a diagram illustrating an example of graph data;
FIG. 3 is a diagram for describing calculation of embedding vectors;
FIG. 4 is a diagram for describing additional data;
FIG. 5 is a functional block diagram of a machine learning device;
FIG. 6 is a diagram for describing a triple representing the additional data;
FIG. 7 is a diagram for describing addition of the additional data to machine-learned graph data;
FIG. 8 is a diagram for describing determination of an online training range in a first embodiment;
FIG. 9 is a block diagram illustrating a schematic configuration of a computer that functions as a machine learning device;
FIG. 10 is a flowchart illustrating an example of machine learning processing;
FIG. 11 is a flowchart illustrating an example of range determination processing in the first embodiment;
FIG. 12 is a flowchart illustrating an example of prediction processing;
FIG. 13 is a diagram for describing determination of an online training range in a second embodiment;
FIG. 14 is a diagram for describing the determination of the online training range in the second embodiment;
FIG. 15 is a flowchart illustrating an example of range determination processing in the second embodiment;
FIG. 16 is a diagram for describing the determination of the online training range in the second embodiment;
FIG. 17 is a diagram for describing an edge to be predicted and additional data;
FIG. 18 is a diagram for describing determination of an online training range in a third embodiment; and
FIG. 19 is a diagram for describing the determination of the online training range in the third embodiment.

### DESCRIPTION OF EMBODIMENTS

### <Machine Learning of Embedding Vectors in Graph Data>

A machine learning device according to each of the following embodiments updates graph data by online training in a case where data is newly acquired for graph data in which embedding vectors have been machine-learned. Hereinafter, graph data in which embedding vectors have been machine-learned is also referred to as "machine-learned graph data". Furthermore, newly acquired data is referred to as "additional data". Before describing details of each embodiment, the machine-learned graph data and a problem of a method generally assumed as the online training of the graph data will be described.

First, the machine-learned graph data will be described taking, as an example, graph data used for prediction of an unintended action (hereinafter, referred to as "side effect") in administration of a medicine. Here, the description will be made assuming that an information processing device generates the machine-learned graph data. The information processing device may be a machine learning device according to each embodiment described below, or may be another computer other than the machine learning device.

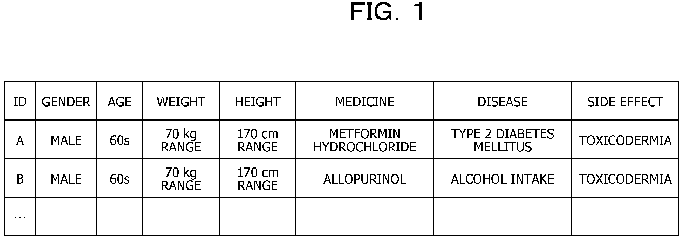

The information processing device generates graph data obtained by converting case data into a graph including nodes and edges coupling between the nodes. In the case of the example here, the case data is data including information such as attributes of patients, administered medicines, and diseases the patients are suffering from, and information regarding side effects. FIG. 1 illustrates an example of the case data. In the example in FIG. 1, information regarding "ID", "gender", "age", "weight", "height", "medicine", "disease", and "side effect" is included for each patient. The "ID" is identification information of a patient. The "gender", the "age", the "weight", and the "height" are examples of attributes of the patient. The "medicine" is a name of a medicine administered to the patient. The "disease" is a name of an underlying disease the patient is suffering from. The "side effect" is information regarding a side effect generated when a medicine indicated in the "medicine" is administered.

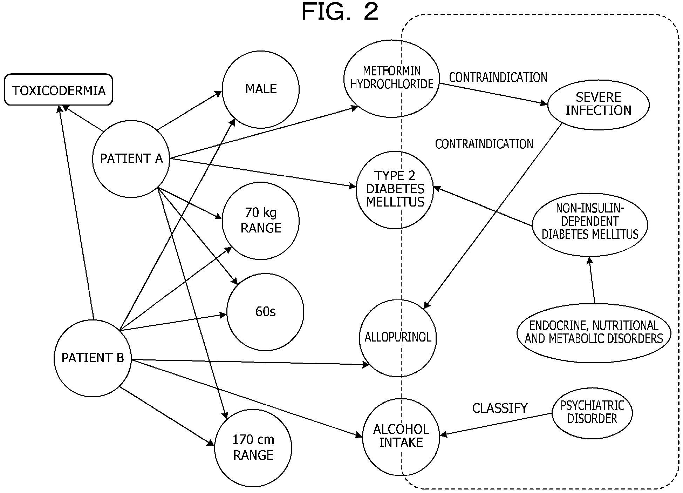

The information processing device generates a node indicating each value of each item included in the case data as described above, and generates graph data by coupling edges from each node of the "ID" to nodes each indicating one of attributes, medicines, diseases, and side effects of a patient indicated by the ID. FIG. 2 illustrates an example of the graph data generated from the case data illustrated in FIG. 1. In FIG. 2, nodes indicated by circles in which the respective values are indicated are the respective nodes indicating attributes, medicines, and diseases, a node indicated by a rounded square in which a side effect is indicated is a node indicating a side effect, and arrows coupling between the nodes are edges.

Furthermore, in the example in FIG. 2, a case where an ontology (a broken line portion in FIG. 2) indicating background knowledge is coupled to the graph data indicating the case data is illustrated. The ontology indicating the background knowledge is a systematization of background knowledge in a concerned field, and here, for example, information such as similarities and relationships between diseases, and similarities between medicines and ingredients included therein is organized in a tree structure format or the like. In the example in FIG. 2, the nodes related to the ontology are represented by ellipses. Conversion of the case data into the graph data facilitates such coupling of the ontology. In a case where diseases are similar, in a case where medicines including the same ingredient are administered, or the like, there is a possibility that similar side effects occur. Therefore, by coupling the ontology to the case data, accuracy of side effect prediction is improved.

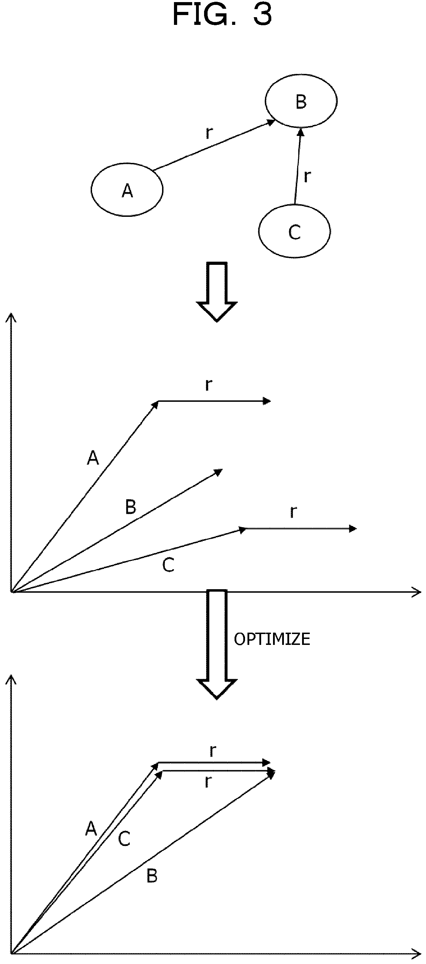

The information processing device calculates embedding vectors representing the respective nodes and the respective edges included in the generated graph data. Specifically, the information processing device calculates the embedding vectors by mapping the respective nodes and edges included in the graph data to an n-dimensional vector space. More specifically, as illustrated in an upper diagram in FIG. 3, calculation of embedding vectors will be described taking, as an example, graph data including nodes A, B, and C, an edge r between the nodes A and B, and an edge r between the nodes C and B. Here, to simplify the description, the case of mapping to a two-dimensional vector space will be described.

First, as illustrated in a middle diagram in FIG. 3, the information processing device arranges the respective nodes and edges included in the graph data in the vector space as initial value vectors. Then, the information processing device optimizes the arrangement of the respective vectors so as to represent coupling relationships between the nodes. In the example in FIG. 3, as illustrated in a lower diagram in FIG. 3, the information processing device optimizes the arrangement of the respective vectors such that a vector A + a vector r is made closer to a vector B, and a vector C + a vector r is made closer to the vector B. The vector after the optimization is an embedding vector of a node indicated by the vector. The graph data in which the embedding vectors for the respective nodes and the respective edges are calculated by the information processing device is the machine-learned graph data.

In a case where additional data is acquired for the graph data as described above, it is needed to update the machine-learned graph data and follow a change represented by the additional data. As a method of updating the graph data, as in the above description, a method of recalculating all the embedding vectors of the respective nodes and the respective edges of the graph data to which the additional data has been added, in other words, a method of executing machine learning by batch processing is considered. However, the machine learning by the batch processing has a high processing load. In particular, in a case where the number of nodes and the number of edges included in the graph data are enormous or in a case where a frequency at which the additional data is acquired is high, a method that more efficiently updates the graph data is desirable.

Thus, a method of online training in which, each time the additional data is acquired, machine learning is executed by using only additional data and a graph is updated is considered. In other words, the information processing device does not recalculate the embedding vectors from the beginning to update the graph data, but executes machine learning with the additional data to update the embedding vectors, which are parameters of the machine-learned graph data, as needed. In this case, there are advantages that a processing load per machine learning is low, it is not needed to store all pieces of data used in the past machine learning execution, and furthermore, it is possible to immediately respond to a data change.

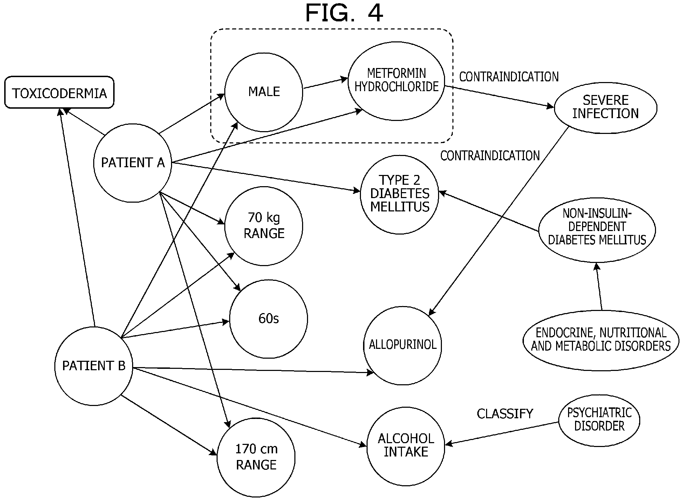

In normal online training, since additional data has all features, all parameters of a machine-learned model may be updated. However, in the online training of the embedding vectors of the graph data, only portions of the nodes and the edges represented by the additional data are changed by the additional data. For example, it is assumed that an edge is added between a node "male" and a node "metformin hydrochloride" as indicated by a broken line portion in FIG. 4 for the machine-learned graph data illustrated in FIG. 2. In this case, only embedding vectors related to a triple (triad) including the two nodes and the edge are updated. In this way, in a case where a range updated by the online training is limited to a range of the additional data, a sufficient change that affects prediction using the graph data may not be obtained. Thus, in each of the following embodiments, the online training is executed after a range in which the online training is executed is determined based on the additional data. With this configuration, each embodiment reduces a processing load as compared with a case of the machine learning by the batch processing while taking advantages of the online training. Hereinafter, examples of embodiments according to the disclosed technology will be described.

### <First Embodiment>

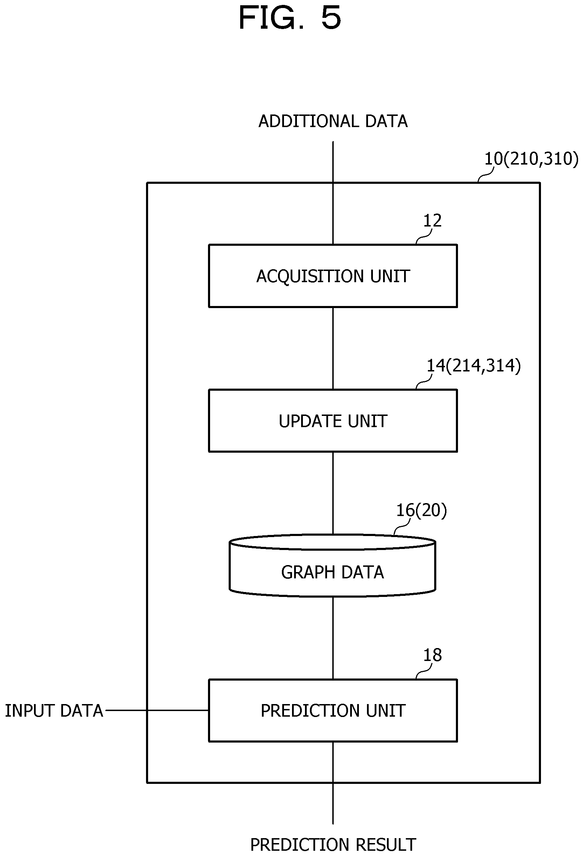

As illustrated in FIG. 5, a machine learning device 10 functionally includes an acquisition unit 12, an update unit 14, and a prediction unit 18. Furthermore, graph data 16 is stored in a predetermined storage area of the machine learning device 10. The graph data 16 is machine-learned graph data in a state before additional data is input and update processing is executed. Note that the graph data subjected to the update processing is assumed to be graph data 20.

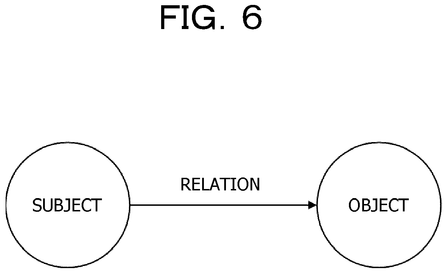

The acquisition unit 12 acquires additional data input to the machine learning device 10. As illustrated in FIG. 6, the additional data is data in a resource description framework (RDF) format, and is information regarding a triple including three elements of two entities and a relation between the entities. Specifically, as illustrated in FIG. 6, the additional data includes a node serving as a subject, a node serving as an object, and an edge serving as a relation between the subject and the object. The node on a starting point side of the edge is the node serving as the subject, and the node on an end point side of the edge is the node serving as the object. The node serving as the subject and the node serving as the object are examples of a "first node" and a "second node" in the disclosed technology, and the edge serving as the relation is an example of a "first edge" in the disclosed technology.
Furthermore, the triple representing the additional data is an example of a "first triple" of the disclosed technology. The acquisition unit 12 transfers the acquired additional data to the update unit 14.

The update unit 14 updates, based on the additional data and a plurality of embedding vectors representing the respective plurality of nodes and the respective plurality of edges of the machine-learned graph data 16, specific embedding vectors among the plurality of embedding vectors. The specific embedding vectors are embedding vectors representing nodes and edges included in a range determined as an online training range. Specifically, the specific embedding vector is an embedding vector of each of one or a plurality of nodes and one or a plurality of edges coupled to the triple representing the additional data under a specific condition in the machine-learned graph data 16. For example, in a case where the triple representing the additional data is added to the machine-learned graph data 16, the update unit 14 may set, as the specific condition, being a triple that is coupled to the triple representing the additional data and that has a distance from the triple representing the additional data a predetermined value or less. The distance here is the number of edges between the nodes. In other words, a distance from a certain node to another node is the number of edges included in a path coupling the two nodes.

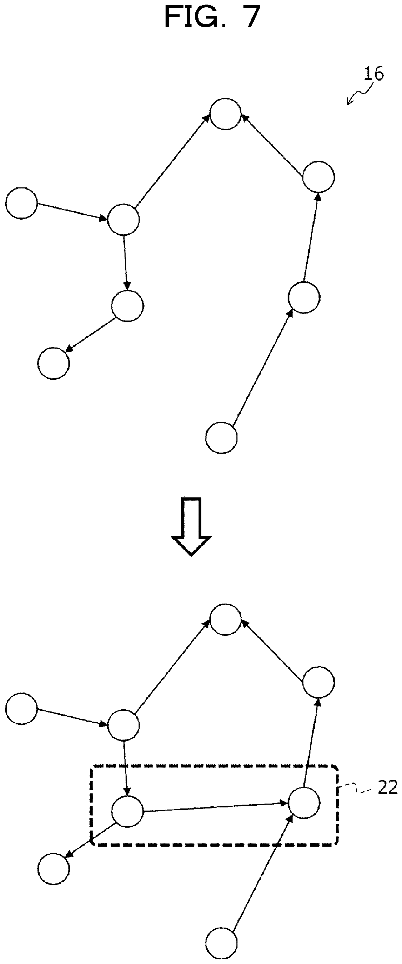

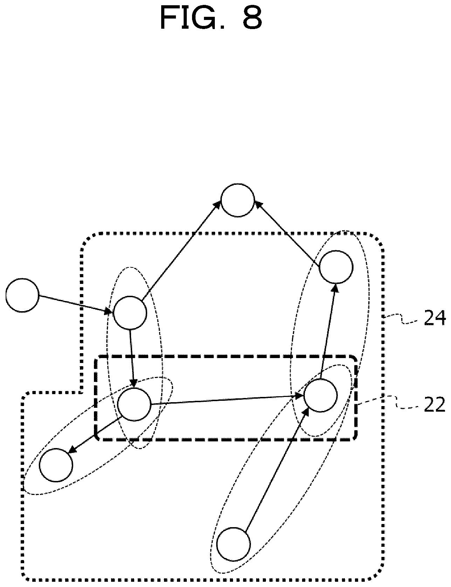

For example, it is assumed that a triple 22 representing additional data as illustrated in a broken line portion of a lower diagram in FIG. 7 is added to the machine-learned graph data 16 as illustrated in an upper diagram in FIG. 7. It is assumed that the predetermined value of the specific condition described above is a distance represented by the number of edges 1. In this case, as illustrated in FIG. 8, the update unit 14 determines, among triples included in the graph data 16, the triple 22 representing the additional data and triples having nodes included in the triple 22 representing the additional data as elements, as an online training range 24. In FIG. 8, the triples surrounded by dotted ellipses are the triples having the nodes included in the triple 22 representing the additional data as the elements. Note that the predetermined value described above is not limited to the distance represented by the number of edges 1, and it is sufficient that the predetermined value described above is appropriately set according to a scale, a type, and the like of the graph data 16.

As described with reference to FIG. 3, the update unit 14 calculates embedding vectors of the respective nodes and edges included in the triples for the respective triples included in the determined online training range 24. With this configuration, the embedding vectors of the nodes and the edges included in the range 24 in the machine-learned graph data 16 are updated, and the updated graph data 20 is obtained.

The prediction unit 18 predicts presence or absence of an edge to be predicted in input data by using the updated graph data 20. The input data is data that does not include an item to be predicted ("side effect" in the example in FIG. 1) in the case data as illustrated in FIG. 1. For example, the prediction unit 18 converts the input data into graph data, and calculates embedding vectors of the respective nodes and edges included in the graph data indicating the input data, as in the above description. Then, the prediction unit 18 specifies a node that is likely to be coupled to the edge to be predicted in the graph data 20 based on similarity between the embedding vectors of the graph data 20 and the embedding vectors of the input data. Then, the prediction unit 18 outputs, as a prediction result, whether or not the edge to be predicted is coupled to the specified node. For example, the prediction unit 18 outputs a prediction result indicating TRUE in a case where the edge to be predicted is coupled to the specified node, and outputs a prediction result indicating FALSE in a case where the edge to be predicted is not coupled to the specified node.

In the case of the example in FIG. 4, edges coupled to a node indicating the side effect "toxicodermia" are edges to be predicted, and the respective nodes indicating "ID" are nodes that are likely to be coupled to the edges to be predicted. Then, the prediction unit 18 predicts whether or not there is a possibility that a patient to be predicted will develop the side effect "toxicodermia" based on presence or absence of an edge coupling the node indicating the "ID" of the specified patient and the node indicating the side effect "toxicodermia".

Note that, as a feature of the prediction using the graph data, the input data does not need to have values of all items other than the item to be predicted included in the case data. This is because the graph data may be generated even when the input data lacks values of a part of the items, and prediction may be performed when similarity between the generated graph data of the input data and a part of the graph data 20 may be determined.

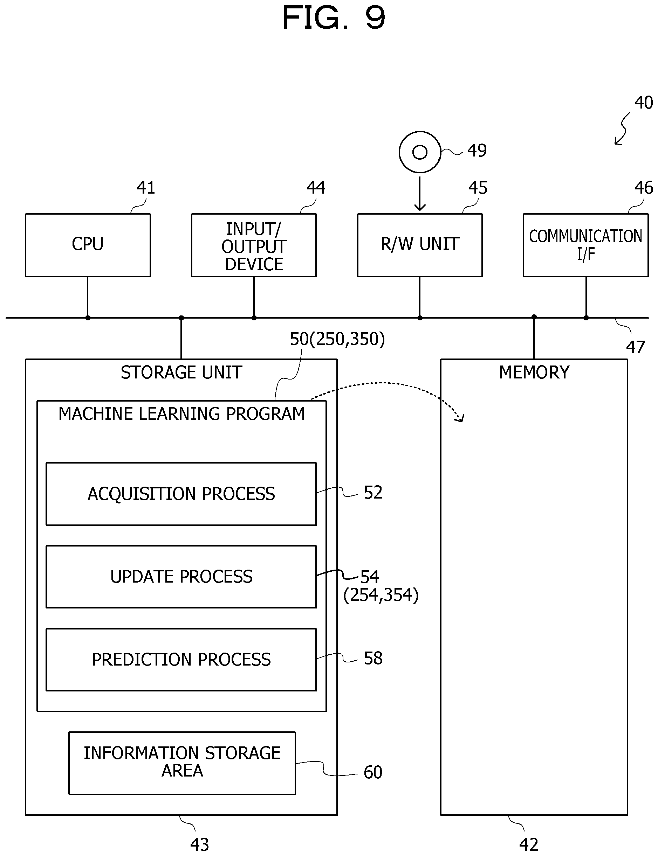

The machine learning device 10 may be implemented by, for example, a computer 40 illustrated in FIG. 9. The computer 40 includes a central processing unit (CPU) 41, a memory 42 as a temporary storage area, and a nonvolatile storage unit 43. Furthermore, the computer 40 includes an input/output device 44 such as an input unit and a display unit, and a read/write (R/W) unit 45 that controls reading and writing of data from and to a storage medium 49. Furthermore, the computer 40 includes a communication interface (I/F) 46 to be coupled to a network such as the Internet. The CPU 41, the memory 42, the storage unit 43, the input/output device 44, the R/W unit 45, and the communication I/F 46 are coupled to each other via a bus 47.

The storage unit 43 may be implemented by a hard disk drive (HDD), a solid state drive (SSD), a flash memory, or the like. The storage unit 43 as a storage medium stores a machine learning program 50 for causing the computer 40 to function as the machine learning device 10. The machine learning program 50 includes an acquisition process 52, an update process 54, and a prediction process 58. Furthermore, the storage unit 43 includes an information storage area 60 for storing information included in the graph data 16 (20).

The CPU 41 reads the machine learning program 50 from the storage unit 43 to load the read machine learning program 50 into the memory 42, and sequentially executes the processes included in the machine learning program 50. The CPU 41 operates as the acquisition unit 12 illustrated in FIG. 5 by executing the acquisition process 52. Furthermore, the CPU 41 operates as the update unit 14 illustrated in FIG. 5 by executing the update process 54. Furthermore, the CPU 41 operates as the prediction unit 18 illustrated in FIG. 5 by executing the prediction process 58. Furthermore, the CPU 41 reads information from the information storage area 60 to load the graph data 16 (20) into the memory 42. With this configuration, the computer 40 that has executed the machine learning program 50 functions as the machine learning device 10. Note that the CPU 41 that executes the program is hardware. Furthermore, the CPU 41 is an example of a "control unit" of the disclosed technology.

Note that functions implemented by the machine learning program 50 may also be implemented by, for example, a semiconductor integrated circuit, more specifically, an application specific integrated circuit (ASIC) or the like.

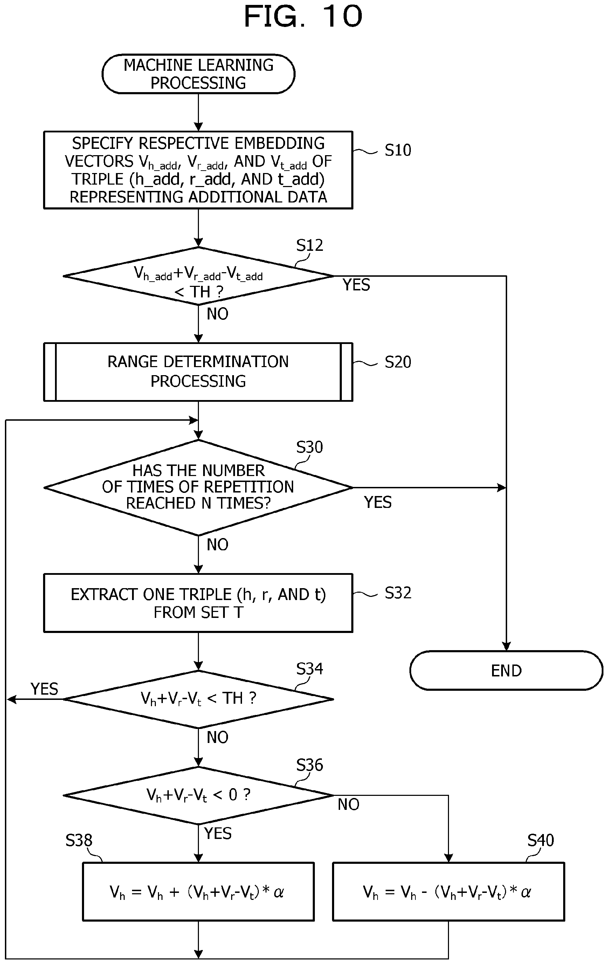

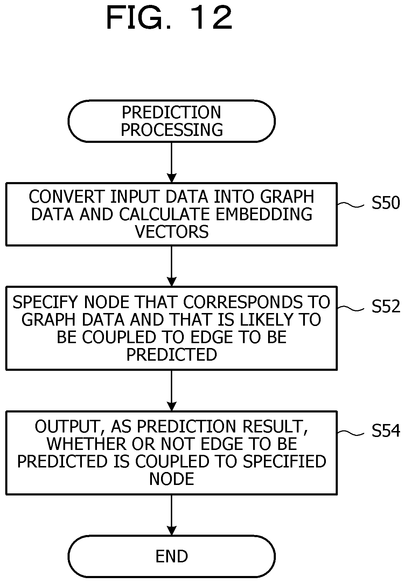

Next, operation of the machine learning device 10 according to a first embodiment will be described. When additional data is input to the machine learning device 10, machine learning processing illustrated in FIG. 10 is executed in the machine learning device 10. When input data is input to the machine learning device 10, prediction processing illustrated in FIG. 12 is executed in the machine learning device 10. Note that the machine learning processing is an example of a machine learning method of the disclosed technology, and the prediction processing is an example of a prediction method of the disclosed technology. Hereinafter, the machine learning processing and the prediction processing will be described in detail.

First, the machine learning processing illustrated in FIG. 10 will be described. In Step S10, the acquisition unit 12 acquires the additional data input to the machine learning device 10 and transfers the acquired additional data to the update unit 14. Then, the update unit 14 specifies the respective embedding vectors V_{h_add}, V_{r_add}, and V_{t_add} of a triple (h_add, r_add, and t_add) representing the additional data. Specifically, in a case where the triple (h_add, r_add, and t_add) is added to the graph data 16, the update unit 14 specifies, as V_{h_add}, an embedding vector for a node of the graph data 16 corresponding to a node h_add serving as a subject. Similarly, the update unit 14 specifies, as V_{t_add}, an embedding vector for a node of the graph data 16 corresponding to a node t_add serving as an object. Furthermore, the update unit 14 specifies, as V_{r_add}, an embedding vector for an edge of the graph data 16 indicating a relation similar to that of an edge r_add.

Next, in Step S12, the update unit 14 determines whether or not V_{h_add} + V_{r_add} - V_{t_add} is smaller than a predetermined threshold TH. As the threshold TH, a value with which it may be determined that V_{h_add} + V_{r_add} and V_{t_add} substantially match is set in advance. In a case where V_{h_add} + V_{r_add} - V_{t_add} < TH is satisfied, the embedding vectors of the graph data 16 do not change even when the additional data is added, and thus, the machine learning processing ends as it is. On the other hand, in a case where V_{h_add} + V_{r_add} - V_{t_add} ≥ TH is satisfied, it is needed to update the embedding vectors of the graph data 16 according to the additional data, and thus the processing proceeds to Step S20.

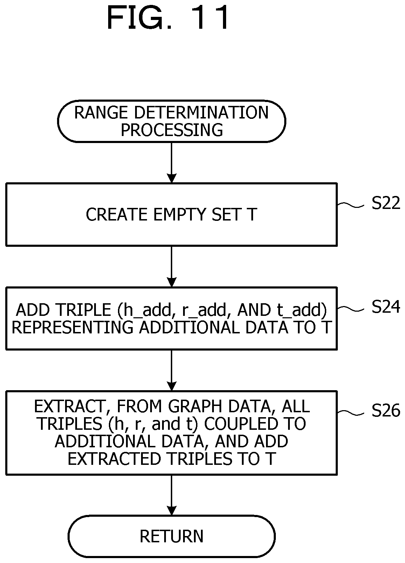

In Step S20, range determination processing is executed. Here, the range determination processing will be described with reference to FIG. 11. Here, a case will be described where a range is determined such that triples directly coupled to the triple representing the additional data (triples whose distances are the number of edges 1) is included in an online training range. In Step S22, the update unit 14 creates an empty set as a set T for storing the triples of the graph data 16 included in the online training range. Next, in Step S24, the update unit 14 adds the triple (h_add, r_add, and t_add) representing the additional data to the set T. Next, in Step S26, the update unit 14 extracts, from the graph data 16, all the triples directly coupled to the triple 22 representing the additional data, in other words, all the triples having nodes included in the triple 22 representing the additional data as elements, and adds the extracted triples to the set T. Hereinafter, the triples added to the set T, including the triple (h_add, r_add, and t_add), will be referred to as (h, r, and t). The update unit 14 randomly provides initial values of embedding vectors to the respective nodes and edges included in the triples added to the set T. Then, the processing returns to the machine learning processing (FIG. 10).

Next, in Step S30, it is determined whether or not the update processing in Steps S32 to S40 described later has been repeated a predetermined number of times (N times). In a case where the number of times of repetition has not reached N times, the processing proceeds to Step S32. In Step S32, the update unit 14 extracts one triple (h, r, and t) from the set T. Next, in Step S34, the update unit 14 determines whether or not Vₕ + Vᵣ - Vₜ < TH is satisfied by using embedding vectors Vₕ, Vᵣ, and Vₜ of the respective elements of the extracted triple (h, r, and t). In a case where Vₕ + Vᵣ - Vt < TH is satisfied, the processing returns to Step S30, and in a case where Vₕ + Vᵣ - Vₜ ≥ TH is satisfied, the processing proceeds to Step S36.

In Step S36, the update unit 14 determines whether or not Vₕ + Vᵣ - Vₜ < 0 is satisfied. In a case where Vₕ + Vᵣ - Vₜ < 0 is satisfied, the processing proceeds to Step S38, and the update unit 14 updates an embedding vector for a node h by adding a value obtained by multiplying (Vₕ + Vᵣ - Vₜ) by a predetermined coefficient α to Vₕ. On the other hand, in a case where Vₕ + Vᵣ - Vₜ ≥ 0 is satisfied, the processing proceeds to Step S40, and the update unit 14 updates the embedding vector for the node h by subtracting the value obtained by multiplying (Vₕ + Vᵣ - Vₜ) by the predetermined coefficient α from Vₕ. Then, the processing returns to Step S30. The processing of repeating Steps S38 and S40 N times corresponds to the optimization in the calculation of the embedding vector described with reference to FIG. 3.

When it is determined in Step S30 that the number of times of repetition has reached N times, the machine learning processing ends. With this configuration, the graph data 20 obtained by updating the embedding vectors of the nodes and the edges in the range determined in the range determination processing in Step S20 described above is generated.

Next, the prediction processing illustrated in FIG. 12 will be described. In Step S50, the prediction unit 18 acquires the input data input to the machine learning device 10, converts the acquired input data into graph data, and calculates embedding vectors of the respective nodes and edges included in the graph data indicating the input data. Next, in Step S52, the prediction unit 18 specifies a node that is likely to be coupled to an edge to be predicted in the graph data 20 based on similarity between the embedding vectors of the graph data 20 and the embedding vectors of the input data. Next, in Step S54, the prediction unit 18 outputs, as a prediction result, whether or not the edge to be predicted is coupled to the specified node, and the prediction processing ends.

As described above, the machine learning device according to the first embodiment includes a plurality of nodes and a plurality of edges indicating relationships between the plurality of nodes, and adds additional data to graph data in which embedding vectors representing the respective nodes and edges are calculated by machine learning. Then, the machine learning device updates the embedding vectors of the graph data. At this time, the machine learning device determines, as an online training range, a range including one or a plurality of nodes and one or a plurality of edges coupled to a triple representing the additional data under a specific condition in the graph data. Then, the machine learning device updates the embedding vectors representing the respective nodes and edges in the determined range. With this configuration, it is possible to reduce a processing load of machine learning of the embedding vectors of the graph data.

### <Second Embodiment>

Next, a second embodiment will be described. Note that, in a machine learning device according to the second embodiment, similar components to those of the machine learning device 10 according to the first embodiment are denoted by the same reference signs, and detailed description thereof will be omitted. Furthermore, in functional configurations for which the last two digits of reference signs are common between the first embodiment and the second embodiment, detailed description of the common functions will be omitted.

As illustrated in FIG. 5, a machine learning device 210 functionally includes an acquisition unit 12, an update unit 214, and a prediction unit 18. Furthermore, graph data 16 (20) is stored in a predetermined storage area of the machine learning device 210.

Similarly to the update unit 14 in the first embodiment, the update unit 214 updates embedding vectors of the graph data 16 based on additional data, and generates the updated graph data 20. Since a method of determining an online training range of the update unit 214 in the second embodiment is different from that of the update unit 14 in the first embodiment, hereinafter, the method of determining the range by the update unit 214 in the second embodiment will be described.

The update unit 214 determines the online training range according to a structure of the graph data 16. For example, the structure of the graph data 16 is different between graph data obtained by converting data in a table format as illustrated in FIG. 1 and graph data representing connection between users in a social networking service (SNS). In the case of the former graph data 16, for example, as illustrated in FIG. 2, there is a strong tendency that edges are coupled from one node indicating "ID" to a plurality of nodes. On the other hand, in the case of the latter graph data 16, there is a strong tendency that edges are coupled from one node to other nodes in a chained manner. Due to such a difference in structure, an influence range when the additional data is added to the graph data 16 is also different. For example, in the case of the former graph data 16, it is considered that an influence of the additional data strongly acts in a direction of a sibling relationship of nodes (between child nodes coupled to the same parent node), and in the case of the latter graph data 16, it is considered that the influence of the additional data strongly acts in a direction of a descendant relationship.

Thus, the update unit 214 determines the structure of the graph data as described above. Specifically, the update unit 214 calculates a ratio of the number of nodes serving as both a subject and an object among the nodes of the graph data 16. In a case where the ratio is small, the structure of the former graph data described above is represented, and in a case where the ratio is large, the structure of the latter graph data described above is represented.

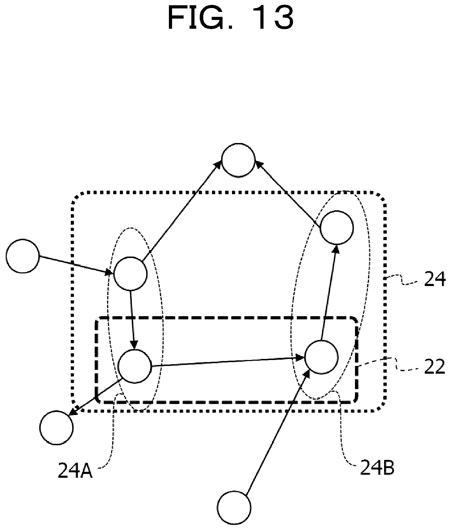

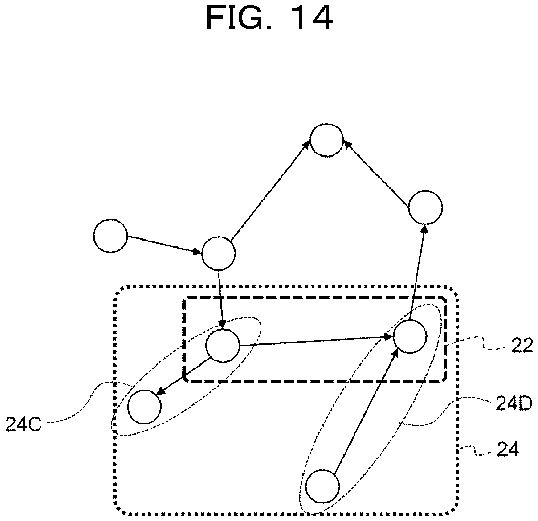

In a case where the calculated ratio is equal to or smaller than a predetermined ratio (for example, 50%), as illustrated in FIG. 13, the update unit 214 determines a range 24 so that a triple 24A that couples, as the object, a node serving as the subject in a triple 22 representing the additional data is included in the range 24. Furthermore, the update unit 214 determines the range 24 so that a triple 24B that couples, as the subject, a node serving as the object in the triple 22 representing the additional data is included in the range 24. In other words, the update unit 214 determines the range 24 so that a relationship between nodes included in the triple 22 representing the additional data and the triples 24A and 24B coupled to the triple 22 becomes a descendant relationship. On the other hand, in a case where the calculated ratio exceeds the predetermined ratio (for example, 50%), as illustrated in FIG. 14, the update unit 214 determines the range 24 so that a triple 24C that couples, as the subject, the node serving as the subject in the triple 22 representing the additional data is included in the range 24. Furthermore, the update unit 214 determines the range 24 so that a triple 24D that couples, as the object, the node serving as the object in the triple 22 representing the additional data is included in the range 24. In other words, the update unit 214 determines the range 24 so that a relationship between nodes included in the triple 22 representing the additional data and the triples 24C and 24D coupled to the triple 22 becomes a sibling relationship with the common node as the center.

The machine learning device 210 may be implemented by, for example, a computer 40 illustrated in FIG. 9. A storage unit 43 of the computer 40 stores a machine learning program 250 for causing the computer 40 to function as the machine learning device 210. The machine learning program 250 includes an acquisition process 52, an update process 254, and a prediction process 58. Furthermore, the storage unit 43 includes an information storage area 60 for storing information included in the graph data 16 (20).

A CPU 41 reads the machine learning program 250 from the storage unit 43 to load the read machine learning program 250 into a memory 42 and sequentially executes the processes included in the machine learning program 250. The CPU 41 operates as the update unit 214 illustrated in FIG. 5 by executing the update process 254. The other processes are similar to those of the machine learning program 50 according to the first embodiment. With this configuration, the computer 40 that has executed the machine learning program 250 functions as the machine learning device 210. Note that the functions implemented by the machine learning program 250 may also be implemented by, for example, a semiconductor integrated circuit, more specifically, an ASIC or the like.

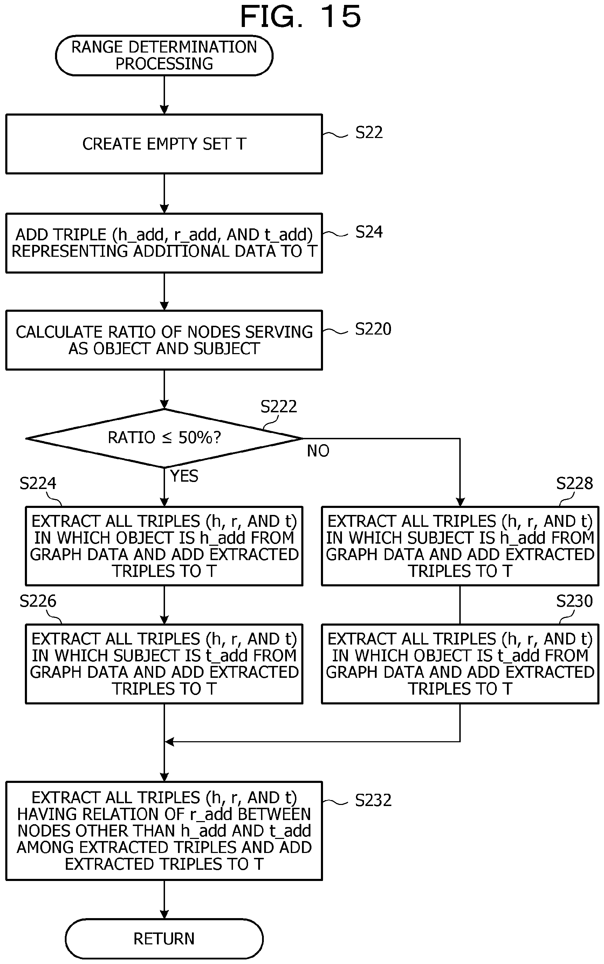

Next, operation of the machine learning device 210 according to the second embodiment will be described. In the second embodiment, range determination processing in machine learning processing is different from that of the first embodiment. Therefore, the range determination processing in the second embodiment will be described with reference to FIG. 15. Note that, in the range determination processing in the second embodiment, similar processing to that of the range determination processing (FIG. 11) in the first embodiment is denoted by the same step number, and detailed description thereof will be omitted.

When the processing proceeds to Step S220 through Steps S22 and S24, the update unit 214 calculates a ratio of the number of nodes serving as both a subject and an object among the nodes of the graph data 16. Next, in Step S222, the update unit 214 determines whether or not the calculated ratio is equal to or smaller than a predetermined ratio (for example, 50%). In a case where the calculated ratio is equal to or smaller than the predetermined ratio, the processing proceeds to Step S224, and in a case where the calculated ratio exceeds the predetermined ratio, the processing proceeds to Step S228.

In Step S224, the update unit 214 extracts all triples (h, r, and t) in which the object is a node h_add (node serving as the subject) from the graph data 16, and adds the extracted triples to the set T. Next, in Step S226, the update unit 214 extracts all triples (h, r, and t) in which the subject is a node t_add (node serving as the object) from the graph data 16, adds the extracted triples to the set T, and the processing proceeds to Step S232.

On the other hand, in Step S228, the update unit 214 extracts all triples (h, r, and t) in which the subject is the node h_add (node serving as the subject) from the graph data 16, and adds the extracted triples to the set T. Next, in Step S230, the update unit 214 extracts all triples (h, r, and t) in which the object is the node t_add (node serving as the object) from the graph data 16, adds the extracted triples to the set T, and the processing proceeds to Step S232.

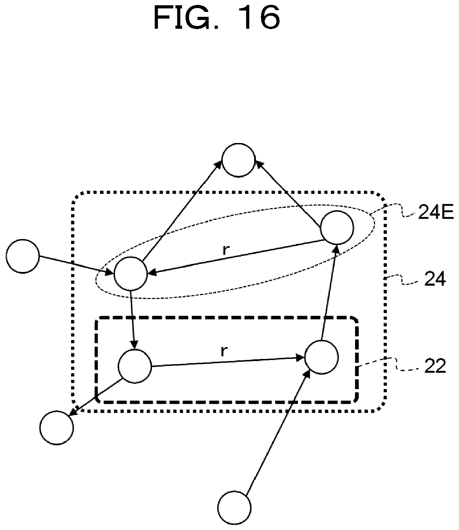

In Step S232, the update unit 214 extracts all triples (h, r, and t) having the same relation as that of r_add between nodes other than the nodes h_add and t_add among the nodes of the extracted triples, and adds the extracted triples to the set T. This includes a triple such as a triple 24E illustrated in FIG. 16 in the range 24. Then, the range determination processing ends, and the processing returns to the machine learning processing (FIG. 10).

As described above, the machine learning device according to the second embodiment determines an online training range according to a ratio of the number of nodes serving as both a subject and an object among nodes of graph data. With this configuration, the online training range may be more appropriately determined according to a structure of the graph data, and a processing load of machine learning of embedding vectors of the graph data may be reduced.

### <Third Embodiment>

Next, a third embodiment will be described. Note that, in a machine learning device according to the third embodiment, similar components to those of the machine learning device 10 according to the first embodiment are denoted by the same reference signs, and detailed description thereof will be omitted. Furthermore, in functional configurations for which the last two digits of reference signs are common between the first embodiment and the third embodiment, detailed description of the common functions will be omitted.

As illustrated in FIG. 5, a machine learning device 310 functionally includes an acquisition unit 12, an update unit 314, and a prediction unit 18. Furthermore, graph data 16 (20) is stored in a predetermined storage area of the machine learning device 310.

Similarly to the update unit 14 in the first embodiment, the update unit 314 updates embedding vectors of the graph data 16 based on additional data, and generates the updated graph data 20. Since a method of determining an online training range of the update unit 314 in the third embodiment is different from that of the update unit 14 in the first embodiment, hereinafter, the method of determining the range by the update unit 314 in the third embodiment will be described.

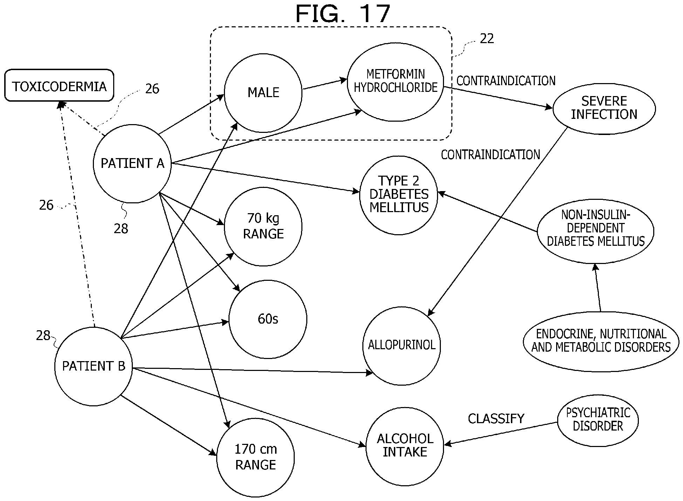

Here, as illustrated in FIG. 17, in a case where presence or absence of an edge 26 to be predicted is predicted in prediction using graph data, it is desirable to determine an online training range so that the prediction is affected by additional data. However, in the method of determining the online training range in the first and second embodiments, for example, in a case where a distance to the edge 26 to be predicted is long from a portion to which a triple representing the additional data is added, or the like, the range may not be determined so as to affect the prediction.

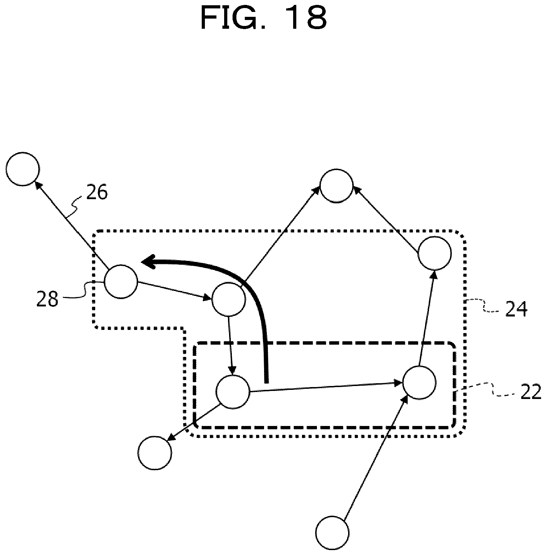

Thus, the update unit 314 determines the range so as to include a path from one node having a shorter distance to a node (hereinafter, referred to as "prediction node") 28 that is likely to be coupled to the edge 26 to be predicted between nodes of a triple 22 representing the additional data to the prediction node 28. For example, in a case where a range 24 is determined as illustrated in FIG. 13, the update unit 314 searches for the shortest path from a node serving as a subject of the triple 22 to the prediction node 28 and the shortest path from a node serving as an object to the prediction node 28, and selects a path with the shorter distance. In this case, as illustrated in FIG. 18, the update unit 314 selects the shortest path from the node serving as the subject to the prediction node 28. As illustrated in FIG. 18, the update unit 314 extends the range 24 so that nodes and edges included in the selected path are included.

The machine learning device 310 may be implemented by, for example, a computer 40 illustrated in FIG. 9. A storage unit 43 of the computer 40 stores a machine learning program 350 for causing the computer 40 to function as the machine learning device 310. The machine learning program 350 includes an acquisition process 52, an update process 354, and a prediction process 58. Furthermore, the storage unit 43 includes an information storage area 60 for storing information included in the graph data 16 (20).

A CPU 41 reads the machine learning program 350 from the storage unit 43 to load the read machine learning program 350 into a memory 42 and sequentially executes the processes included in the machine learning program 350. The CPU 41 operates as the update unit 314 illustrated in FIG. 5 by executing the update process 354. The other processes are similar to those of the machine learning program 50 according to the first embodiment. With this configuration, the computer 40 that has executed the machine learning program 350 functions as the machine learning device 310. Note that the functions implemented by the machine learning program 350 may also be implemented by, for example, a semiconductor integrated circuit, more specifically, an ASIC or the like.

Regarding operation of the machine learning device 310 according to the third embodiment, since only range determination processing in machine learning processing is different from the range determination processing (FIGs. 11 and 15) in the first and second embodiments, detailed description thereof will be omitted. Specifically, the difference is that the update unit 314 determines the range so that the range determined in the range determination processing in the first and second embodiments includes nodes and edges from the triple 22 representing the additional data to the prediction node 28.

As described above, the machine learning device according to the third embodiment determines a range so as to include nodes and edges from a triple representing additional data to a prediction node. With this configuration, an influence of the additional data may be exerted on prediction.

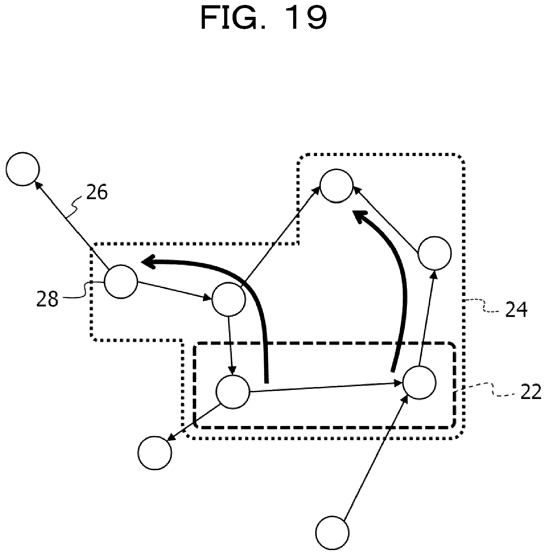

Note that, in the third embodiment, the case has been described where the range is extended so as to include a path from one node of the triple representing the additional data to the prediction node, but the range may also be extended on a side of another node. Specifically, the update unit may determine the range so as to include nodes and edges included from the another node of the triple representing the additional data to a node having the same distance as a distance from the one node to the prediction node. For example, in the example in FIG. 18, the range is determined so as to include the path from the node serving as the subject to the prediction node, but a length (corresponding to the distance) of the path is 2. Therefore, as illustrated in FIG. 19, the update unit may extend the range 24 so as to also include edges and nodes included in a path having a length of 2 from the node serving as the object. With this configuration, it is possible to eliminate imbalance of the range to be subjected to online training between the side of the node serving as the subject of the triple representing the additional data and the side of the node serving as the object.

Note that, in each of the embodiments described above, the case has been described where the machine learning processing and the prediction processing are performed by one computer, but the disclosed technology is not limited to this. The machine learning device including the acquisition unit and the update unit and a prediction device including the prediction unit may be implemented by different computers from each other. Furthermore, in this case, the storage unit of the machine learning device stores the machine learning program for executing the machine learning processing in each of the embodiments described above. Furthermore, a storage unit of the prediction device stores a prediction program for executing the prediction processing in each of the embodiments described above.

Furthermore, an aspect in which the machine learning program is stored (installed) in the storage unit in advance has been described in each of the embodiments described above, but the disclosed technology is not limited to this. The program according to the disclosed technology may also be provided in a form stored in a storage medium such as a CD-ROM, a DVD-ROM, or a USB memory.

### REFERENCE SIGNS LIST

10, 210, 310Machine learning device
12Acquisition unit
14, 214, 314Update unit
16Machine-learned graph data
18Prediction unit
20Updated graph data
22Triple representing additional data
24Online training range
24A, 24B, 24C, 24D, 24ETriple included in online training range
26Edge to be predicted
28Prediction node
40Computer
41CPU
42Memory
43Storage unit
49Storage medium
50, 250, 350Machine learning program

## Claims

1. A machine learning program that causes a computer to execute a process, the process comprising:
acquiring information regarding a first triple that includes a first node, a second node, and a first edge that indicates a relationship between the first node and the second node; and
updating, based on the information and a plurality of vectors that represents respective plurality of nodes and respective plurality of edges that indicate relationships between the plurality of nodes, which is generated by machine learning that uses the plurality of nodes and the plurality of edges, respective vectors of one or a plurality of nodes and one or a plurality of edges that are coupled to the first triple under a specific condition among the plurality of vectors.

2. The machine learning program according to claim 1, wherein
the specific condition includes that, in a case where the first triple is added to graph data that includes the plurality of nodes and the plurality of edges, a distance from the first triple, which is represented by the number of edges between the first triple and each of the one or the plurality of nodes, is equal to or smaller than a predetermined value.

3. The machine learning program according to claim 2, wherein
the predetermined value is a distance represented by the number of edges 1.

4. The machine learning program according to claim 3, wherein,
in a case where a node on a starting point side of an edge is a subject and a node on an end point side of an edge is an object, and a ratio of the number of nodes that serve as both a subject and an object among the plurality of nodes is equal to or smaller than a predetermined ratio,
the specific condition includes that a node that serves as a subject in the first triple is coupled as an object and a node that serves as an object in the first triple is coupled as a subject.

5. The machine learning program according to claim 3, wherein,
in a case where a node on a starting point side of an edge is a subject and a node on an end point side of an edge is an object, and a ratio of the number of nodes that serve as both a subject and an object among the plurality of nodes exceeds a predetermined ratio,
the specific condition includes that a node that serves as a subject in the first triple is coupled as a subject and a node that serves as an object in the first triple is coupled as an object.

6. The machine learning program according to any one of claims 2 to 5, wherein
the specific condition includes to be a node and an edge included from one of the first node and the second node that has a shorter distance to a prediction node coupled to an edge to be predicted at a time of prediction that uses the graph data to the prediction node.

7. The machine learning program according to claim 6, wherein
the specific condition includes to be a node and an edge included from another node of the first node and the second node to a node that has the same distance as the distance from the one node to the prediction node.

8. A prediction program that causes a computer to execute a process, the process comprising:
acquiring input data to be predicted; and
predicting presence or absence of an edge to be predicted in the input data based on information regarding a first triple that includes a first node, a second node, and a first edge that indicates a relationship between the first node and the second node and a plurality of vectors that represents respective plurality of nodes and respective plurality of edges that indicate relationships between the plurality of nodes, which is generated by machine learning that uses the plurality of nodes and the plurality of edges, by using graph data that includes the plurality of nodes and the plurality of edges, in which respective vectors of one or a plurality of nodes and one or a plurality of edges that are coupled to the first triple under a specific condition among the plurality of vectors is updated.

9. A machine learning device comprising a control unit that executes processing including:
acquiring information regarding a first triple that includes a first node, a second node, and a first edge that indicates a relationship between the first node and the second node; and
updating, based on the information and a plurality of vectors that represents respective plurality of nodes and respective plurality of edges that indicate relationships between the plurality of nodes, which is generated by machine learning that uses the plurality of nodes and the plurality of edges, respective vectors of one or a plurality of nodes and one or a plurality of edges that are coupled to the first triple under a specific condition among the plurality of vectors.

10. The machine learning device according to claim 9, wherein
the specific condition includes that, in a case where the first triple is added to graph data that includes the plurality of nodes and the plurality of edges, a distance from the first triple, which is represented by the number of edges between the first triple and each of the one or the plurality of nodes, is equal to or smaller than a predetermined value.

11. The machine learning device according to claim 10, wherein
the predetermined value is a distance represented by the number of edges 1.

12. The machine learning device according to claim 11, wherein,
in a case where a node on a starting point side of an edge is a subject and a node on an end point side of an edge is an object, and a ratio of the number of nodes that serve as both a subject and an object among the plurality of nodes is equal to or smaller than a predetermined ratio,
the specific condition includes that a node that serves as a subject in the first triple is coupled as an object and a node that serves as an object in the first triple is coupled as a subject.

13. The machine learning device according to claim 11, wherein,
in a case where a node on a starting point side of an edge is a subject and a node on an end point side of an edge is an object, and a ratio of the number of nodes that serve as both a subject and an object among the plurality of nodes exceeds a predetermined ratio,
the specific condition includes that a node that serves as a subject in the first triple is coupled as a subject and a node that serves as an object in the first triple is coupled as an object.

14. The machine learning device according to any one of claims 10 to 13, wherein
the specific condition includes to be a node and an edge included from one of the first node and the second node that has a shorter distance to a prediction node coupled to an edge to be predicted at a time of prediction that uses the graph data to the prediction node.

15. The machine learning device according to claim 14, wherein
the specific condition includes to be a node and an edge included from another node of the first node and the second node to a node that has the same distance as the distance from the one node to the prediction node.

16. A prediction device comprising a control unit that executes processing including:
acquiring input data to be predicted; and
predicting presence or absence of an edge to be predicted in the input data based on information regarding a first triple that includes a first node, a second node, and a first edge that indicates a relationship between the first node and the second node and a plurality of vectors that represents respective plurality of nodes and respective plurality of edges that indicate relationships between the plurality of nodes, which is generated by machine learning that uses the plurality of nodes and the plurality of edges, by using graph data that includes the plurality of nodes and the plurality of edges, in which respective vectors of one or a plurality of nodes and one or a plurality of edges that are coupled to the first triple under a specific condition among the plurality of vectors is updated.

17. A machine learning method executed by a computer, the machine learning method comprising:
acquiring information regarding a first triple that includes a first node, a second node, and a first edge that indicates a relationship between the first node and the second node; and
updating, based on the information and a plurality of vectors that represents respective plurality of nodes and respective plurality of edges that indicate relationships between the plurality of nodes, which is generated by machine learning that uses the plurality of nodes and the plurality of edges, respective vectors of one or a plurality of nodes and one or a plurality of edges that are coupled to the first triple under a specific condition among the plurality of vectors.

18. A prediction method executed by a computer, the prediction method comprising:
acquiring input data to be predicted; and
predicting presence or absence of an edge to be predicted in the input data based on information regarding a first triple that includes a first node, a second node, and a first edge that indicates a relationship between the first node and the second node and a plurality of vectors that represents respective plurality of nodes and respective plurality of edges that indicate relationships between the plurality of nodes, which is generated by machine learning that uses the plurality of nodes and the plurality of edges, by using graph data that includes the plurality of nodes and the plurality of edges, in which respective vectors of one or a plurality of nodes and one or a plurality of edges that are coupled to the first triple under a specific condition among the plurality of vectors is updated.

19. A storage medium storing a machine learning program that causes a computer to execute a process, the process comprising:
acquiring information regarding a first triple that includes a first node, a second node, and a first edge that indicates a relationship between the first node and the second node; and
updating, based on the information and a plurality of vectors that represents respective plurality of nodes and respective plurality of edges that indicate relationships between the plurality of nodes, which is generated by machine learning that uses the plurality of nodes and the plurality of edges, respective vectors of one or a plurality of nodes and one or a plurality of edges that are coupled to the first triple under a specific condition among the plurality of vectors.

20. A storage medium storing a prediction program that causes a computer to execute a process, the process comprising:
acquiring input data to be predicted; and
predicting presence or absence of an edge to be predicted in the input data based on information regarding a first triple that includes a first node, a second node, and a first edge that indicates a relationship between the first node and the second node and a plurality of vectors that represents respective plurality of nodes and respective plurality of edges that indicate relationships between the plurality of nodes, which is generated by machine learning that uses the plurality of nodes and the plurality of edges, by using graph data that includes the plurality of nodes and the plurality of edges, in which respective vectors of one or a plurality of nodes and one or a plurality of edges that are coupled to the first triple under a specific condition among the plurality of vectors is updated.
